(19) Europäisches Patentamt
European Patent Office
Office européen des brevets

(11) **EP 3 049 797 B1**

(12) **EUROPÄISCHE PATENTSCHRIFT**

(45) Veröffentlichungstag und Bekanntmachung des Hinweises auf die Patenterteilung:
**01.05.2024   Patentblatt 2024/18**

(21) Anmeldenummer: **14786140.5**

(22) Anmeldetag: **24.09.2014**

(51) Internationale Patentklassifikation (IPC):
*G01N 25/18* (2006.01)     *G01N 27/16* (2006.01)
*G01N 33/00* (2006.01)

(52) Gemeinsame Patentklassifikation (CPC):
**G01N 25/18; G01N 27/16; G01N 33/005**

(86) Internationale Anmeldenummer:
**PCT/EP2014/070408**

(87) Internationale Veröffentlichungsnummer:
**WO 2015/044228 (02.04.2015 Gazette 2015/13)**

(54) **VERFAHREN ZUR QUANTITATIVEN ANALYSE DER ZUSAMMENSETZUNG EINES GASGEMISCHS UND ZUGEHÖRIGE MESSVORRICHTUNG**

METHOD FOR QUANTITATIVE ANALYSIS OF THE COMPOSITION OF A GAS MIXTURE AND ASSOCIATED MEASURING DEVICE

PROCÉDÉ D'ANALYSE QUANTITATIVE DE LA COMPOSITION D'UN MÉLANGE GAZEUX ET DISPOSITIF DE MESURE CORRESPONDANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.09.2013   DE 102013219294**

(43) Veröffentlichungstag der Anmeldung:
**03.08.2016   Patentblatt 2016/31**

(73) Patentinhaber: **Framatome GmbH**
**91052 Erlangen (DE)**

(72) Erfinder: **HILL, Axel**
**64589 Stockstadt (DE)**

(74) Vertreter: **Lavoix**
**Bayerstraße 83**
**80335 München (DE)**

(56) Entgegenhaltungen:
CH-A- 318 487          DE-A1-102004 060 103
DE-A1-102006 033 160   JP-A- S5 770 435
US-A1- 2009 035 184

EP 3 049 797 B1

**Beschreibung**

Die Erfindung betrifft ein Verfahren zur quantitativen Analyse der Zusammensetzung eines Gasgemisches der Atmosphäre eines Containments einer kerntechnischen Anlage. Die Erfindung betrifft weiterhin eine zur Durchführung des Verfahrens geeignete Vorrichtung.

In kerntechnischen Einrichtungen mit eingelagertem Kernbrennstoff (z. B. Kernkraftwerk, Aufbereitungsanlage, Zwischenlager) muss bei Stör- oder Unfallsituationen abhängig vom jeweiligen Störfall und von gegebenenfalls eingeleiteten Gegenmaßnahmen mit einer möglicherweise signifikanten Freisetzung von Wasserstoff gerechnet werden. Hierbei kann es bei einer unkontrollierten Reaktion mit vorhandenem Sauerstoff zu einer Deflagration oder gar Detonation kommen, die zu einer Gefährdung der Hülle bzw. des Containments führt.

Insbesondere bei vergleichsweise schweren Störfallen mit möglicherweise eintretender Kernschmelze können Wasserstofffreisetzungen innerhalb des Containments in besonders hohen Konzentrationen(z. B. 30 Vol.-%) auftreten, so dass es beim Vorhandensein hoher Undichtigkeiten der Containmenthülle auch zu signifikanten Freisetzungen dieses Wasserstoffs in das umliegenden Reaktorgebäude kommen kann, wie dies beim Störfall in Fukushima geschehen ist.

Für die Überwachung der zum Erhalt der Containment-Integrität vorhandenen Schutzmaßnahmen (z. B. die Wirkung von katalytischen Rekombinatoren) sowie vor der Einleitung von "Severe-Accident" Maßnahmen (z. B. Inertisierungsmaßnahmen und/oder Containment-Venting) ist die Kenntnis der relevanten Parameter zur Brennbarkeit der Containment-Atmosphäre und der angrenzenden Gebäudeteile notwendig.

Insbesondere bei inertisierten Containments (in der Regel Siedewasserreaktor-Containments) ist zur Beurteilung des Zustandes und zur eventuell erforderlichen gezielten Einleitung von Maßnahmen neben der Wasserstoffkonzentration die Erhaltung des Inertzustandes und somit die gleichzeitige Kenntnis der Entwicklung der Sauerstoffkonzentration von Bedeutung.

Grundsätzlich ist die frühe Erkennung einer $H_2$-Freisetzung in die Containmentatmosphäre für die Bewertung des Unfallverlaufes und die Planung der einzuleitenden Maßnahmen wichtig. Damit ist im unteren Messbereich (bis zur unteren Zündgrenze) eine höhere Messgenauigkeit als im Störfallweitbereich (> 10 Vol.-%) erforderlich.

Die $H_2$-Messung hat aus diesen Gründen eine sicherheitsrelevante Bedeutung und wird üblicherweise redundant (d .h. einzelfehlerfest) ausgeführt.

Bisherige Messsysteme zur Beurteilung der Containmentatmosphäre bzw. des Unfallfortschritts benötigen für die $H_2$-Messung ein aufwendiges Probenahmesystem mit Gebäudeabsperrarmaturen, sowie ein aufwendiges Gasaufbereitungssystem zur Wasserdampfkondensation und Kondensatabführung. Für die Messung der $H_2$ und $O_2$-Konzen- tration sind zwei separate Sensoren mit jeweils dezidierter Funktionalität und entsprechende Elektronikeinheiten zur Datenaufbereitung erforderlich.

DE 10 2006 033160 A1 beschreibt eine Sensoranordnung zur Detektion von Gasen, insbesondere von Wasserstoff.

Die relativ großen außerhalb des Containments geführten Probenströme und deren Aktivität erschweren die radiologische Qualifikation der Sensoren und deren Elektronik, oder machen deren Qualifikation für diesen Einsatz gänzlich unmöglich.

Weiterhin besitzen diese Systeme einen hohen elektrischen Energiebedarf und/oder benötigen Kühlwasser zur Kondensation des Wasserdampfes. Die Energieversorgung ist jedoch insbesondere im zu postulierendem Fall von"Station Blackout" problematisch bzw. nicht sichergestellt.

Besonders nachteilig ist, dass durch die Kondensation des Wasserdampfes auf die wahre Konzentration der $H_2$ oder $O_2$-Konzentration rückgerechnet werden muss, also nur eine mittelbare Bestimmung möglich ist. Es handelt sich somit nicht mehr um eine direkte Messung. Für die Bestimmung / Berechnung der real herrschenden Konzentration über diese Methodik sind weitere Druck- und Temperaturmessungen notwendig. JPS5770435A offenbart die Messung der Konzentrationen von Wasserstoff und Sauerstoff in der Atmosphäre eines Containments einer kerntechnischen Anlage wobei eine Reaktionswärme zwischen Sauerstoff und Wasserstoff gemessen wird und nach der Reaktion weiterer Sauerstoff oder Wasserstoff zugefügt wird und eine weitere Reaktionswärme bestimmt wird.

Der vorliegenden Erfindung liegt die Aufgabe zugrunde, ein Verfahren zur quantitativen Analyse der Zusammensetzung eines Gasgemischs anzugeben, das die genannten Nachteile vermeidet. Insbesondere soll auch unter schwierigen Bedingungen, wie sie bei einem schweren Störfall in einer kerntechnischen Anlage herrschen, eine einfache, zuverlässige und möglichst direkte Bestimmung von physikalischen Parametern ermöglicht werden, die das Zündfeld und das Verbrennungsverhalten eines Gasgemisches charakterisieren.

Es soll weiterhin eine zur Durchführung des Verfahrens geeignete Vorrichtung angeben werden.

In Bezug auf das Verfahren wird die Aufgabe erfindungsgemäß gelöst durch die Merkmale des Anspruchs 1. In Bezug auf die Vorrichtung wird die Aufgabe gelöst durch die Merkmale des Anspruchs 7.

Das erfindungsgemäße Konzept unterscheidet sich von dem gegenwärtigen Stand der Technik, indem es zwei Messverfahren auf Basis einer Widerstandsmessung einer Wheatstone'schen Brückenschaltung integriert, d. h. eine redundante und diversitäre $H_2$-Messung ermöglicht, die zusätzlich eine gleichzeitige Messung / Bilanzierung des $O_2$-Ge-

haltes erlaubt.

Eine Messbrücke arbeitet nach dem katalytischen Wärmetönungsprinzip (WT-Detektor), und die andere Messbrücke arbeitet nach dem Wärmeleitfähigkeitsprinzip (WL-Detektor). Die Signale des Wärmeleitdetektors und des Wärmetönungsdetektors werden zur Verstärkung und Auswertung zu einer Auswerteelektronik geleitet. Eine Elektronik kann mehrere Sensoren verarbeiten.

Beide radiologisch unempfindlichen Messkammern befinden sich bevorzugt in einem druck- und temperaturfesten Sensorgehäuse und sind erdbebenfest miteinander verbaut. Die Messkammern sind über eine Membran, die aus einem Sintermetall bestehen kann, mit der Containmentatmosphäre verbunden. Die zu messenden brennbaren Gase Wasserstoff (alternativ Kohlenmonoxid) sowie Sauerstoff werden überwiegend durch Diffusion in die Messzellen transportiert.

Zusätzlich befindet sich am Ort der Messung ein Temperatursensor, etwa auf Basis einer Widerstandstemperaturmessung oder einer Thermospannungsmessung, der z. B. zur Bestimmung des Dampfgehaltes unter der Annahme von Sattdampfbedingungen verwendet werden kann.

Die Messung der Wasserstoffkonzentration wird vorzugsweise in diversitärredundanter Weise von dem WL-Detektor und dem WT-Detektor durchgeführt.

Im unteren Messbereich bis zur unteren Zündgrenze erfolgt die Messung durch das WT-Verfahren mit einer für beginnende Störfälle erforderlichen vergleichsweise hohen Messgenauigkeit (z. B. +/- 0,25 Vol.-%).

Bei einem Sauerstoffgehalt der Atmosphäre unterhalb der stöchiometrischen Zusammensetzung eines $H_2/O_2$-Gemisches werden die Messwerte der Wheatsto-ne'schen Brückenschaltung des WT-Detektors mit einer Kalibrierkurve für die Sauerstoffmessung ausgewertet. Die $O_2$-Konzentration kann somit direkt gemessen werden.

Ergibt ein Vergleich, dass die Signale beider Messbrücken gleich sind (unter Berücksichtigung der Messgenauigkeit / Toleranz), kann die $O_2$-Konzentration in vorteilhafter Weise indirekt aus der Messung der Gesamtwärmeleitfähigkeit des Messgases mit Hilfe des WL-Detektors ermittelt werden. Dazu werden plausible Annahmen für die Anteile der Einzelleitfähigkeiten der Atmosphärenzusammensetzung im Containment zugrunde gelegt. Da die anfängliche Luftkonzentration bekannt ist, kann die $N_2$-Konzentration aus der $H_2$-Messung und der Dampfkonzentration bestimmt werden. Die Dampfkonzentration kann unter der Annahme von Sattdampfbedingungen aus der Temperaturmessung bestimmt werden. Im Ergebnis hängt der Fehler der $O_2$-Bestimmung hierbei im Wesentlichen vom Fehler der $H_2$-Messung ab und ist umso kleiner, je genauer der $H_2$-Gehalt gemessen wird.

Wesentliche Vorteile des erfindungsgemäßen Konzepts gegenüber dem Stand der Technik lassen sich wie folgt zusammenfassen:

- redundante und diversitäre Wasserstoffmessung in einem Sensorkopf
- kombinierte $H_2$-Deflagrationsbereichs- (0-10 Vol.-%) sowie Weitbereichsmessung (bis zu 100 Vol.-%)
- kein Sauerstoff für die $H_2$-Messung erforderlich (wichtig insbesondere für inertisierte Containments)
- hohe Genauigkeit im Bereich der unteren Zündgrenze (bei 4 Vol.-% z. B. +/-0,25 Vol.-%)
- gleichzeitige Messung und/oder Bilanzierung der $O_2$-Konzentration
- damit Beurteilung des Zündfeldes und des Verbrennungsverhaltens möglich (Deflagration, Detonation)
- direkte $H_2/O_2$-Messung in inertisierten Containments mit $O_2$-Konzentration < 5 Vol.-% und $H_2$-Überschuss
- in-situ-Messung
- keine Aktivitätsführung außerhalb Containment erforderlich
- keine Containment-Absperrarmaturen erforderlich
- keine Messgasaufbereitung erforderlich
- hohe radiologische Widerstandsfähigkeit, da keine organischen Teile eingesetzt werden müssen
- keine anfällige Elektronik im "harsh environment" (Containment)
- niedriger Energieverbrauch (< 500 W), der bei "Station Blackout" durch eine autarke Batterieeinheit versorgt werden kann
- geringere Ausfallwahrscheinlichkeit
- schnellere Messzyklen (Online-Messung)
- geringerer Platzbedarf
- weniger komplexe Messtechnik
- geringere Investmentkosten
- einfachere Nachrüstbarkeit
- einfache Kalibrierung

Ein Ausführungsbeispiel der Erfindung wird nachfolgend anhand von Zeichnungen näher erläutert. Darin zeigen in stark vereinfachter und schematischer Darstellung:

FIG. 1     ein Messsystem zur Messung der Konzentrationen von in einem Gasgemisch enthaltenen Gasbestandteilen,

**EP 3 049 797 B1**

hier Wasserstoff und Sauerstoff in der Atmosphäre in einem Containment eines Kernkraftwerks,

FIG. 2    ein Prinzipschaltbild einer besonders vorteilhaften Ausführungsform einer in dem Messsystem gemäß FIG. 1 einsetzbaren Widerstands-Brückenschaltung, und

FIG. 3    ein Diagramm mit Kalibrierkurven für einen Wärmetönungssensor des Messsystems gemäß FIG. 1.

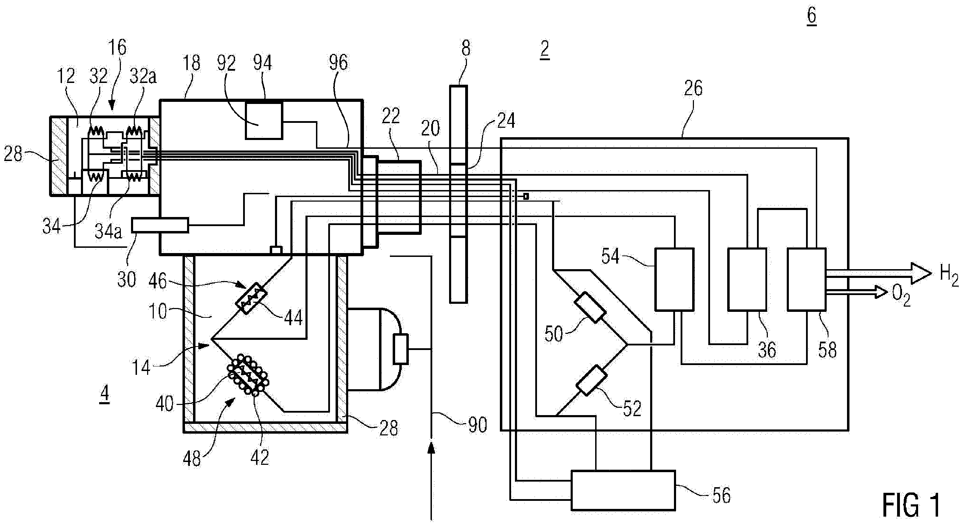

Die in FIG. 1 in einem schematischen Überblick dargestellte Messvorrichtung 2 dient in erster Linie der simultanen Bestimmung des Gehalts von Wasserstoff ($H_2$) und Sauerstoff ($O_2$) in einem Messgas, insbesondere in der Atmosphäre eines auch als Containment 4 bezeichneten Sicherheitseinschlusses in einem Kernkraftwerk 6. Die auch als Sicherheitshülle 8 bezeichnete Containmenthülle selber ist hier nur ausschnittsweise dargestellt.

Die Messvorrichtung 2 umfasst dazu zwei in unmittelbarer räumlicher Nähe zueinander innerhalb des Containments 4 angeordnete Messkammern 10, 12. Das bedeutet, dass der Abstand der beiden Messkammern 10, 12 in Relation zur Ausdehnung des Containments 4 so klein ist, insbesondere weniger als 10 cm, dass man von einer lokalisierten Messung an einem räumlichen Messpunkt sprechen kann.

Eine der Messkammern, nämlich die Messkammer 10 bildet einen Sensorkopf eines katalytischen Wärmetönungsdetektors, kurz WT-Detektor 14 oder WTD, der nach dem Prinzip der katalytischen Verbrennung oder Rekombination arbeitet. Die andere Messkammer 12 bildet einen Sensorkopf eines Wärmeleitfähigkeitsdetektors, kurz WL-Detektor 16 oder WLD (oder TCD nach der englischen Bezeichnung Thermal Conductivity Detector).

Die beiden Messkammern 10, 12 sind im Ausführungsbeispiel an einer gemeinsamen elektrischen Anschlusskammer 18 befestigt, von der ausgehend die elektrischen Anschlussleitungen 20 (Signalleitungen und Stromversorgungsleitungen) über einen störfallfesten Anschlussstecker 22 und eine druckfeste Kabeldurchführung 24 in der Sicherheitshülle 8 zu einer außerhalb des Containments 4 angeordneten elektronischen Auswerteinheit 26 mit integrierter oder externer Stromversorgung geführt sind. Die durch das Containment 4 geführten Anschlussleitungen 20 sind beispielsweise durch mineral-isolierte Kabel verwirklicht. Die Aufteilung ist zweckmäßigerweise so gewählt, dass in den Messkammern 10, 12 innerhalb des Containments 4 nur die zur Verwirklichung des Messprinzips erforderlichen und vergleichsweise strahlenfesten und robusten elektrischen Messzellen angeordnet sind, während die empfindlicheren Komponenten der Auswertungselektronik sich außerhalb des Containments 4 befinden.

Die radiologisch unempfindlichen Messzellen beider Messkammern 10, 12 befinden sich jeweils in einem druck- und temperaturfesten Gehäuse und sind erdbebenfest miteinander verbaut. Die Messkammern 10, 12 sind jeweils über eine Membran 28, die aus einem Sintermetall bestehen kann, strömungsmäßig mit der Containmentatmosphäre verbunden. Die zu messenden Gase werden überwiegend durch Diffusion in die Messkammern 10, 12 transportiert.

In unmittelbarer räumlicher Nähe zu dem WT-Detektor 14 und dem WL-Detektor 16 ist außerdem ein mit dem Messgas beaufschlagter radiologisch robuster Temperatursensor 30 innerhalb des Containments 4 angeordnet, dessen Anschlussleitungen ebenfalls über die Anschlusskammer 18 und die Kabeldurchführung 24 in der Sicherheitshülle 8 zu der elektronischen Auswerteinheit 26 geführt sind. Der Temperatursensor 30 wird beispielsweise durch ein Thermoelement gebildet.

In einer nicht dargestellten Abwandlung können der WT-Detektor 14 und der WL-Detektor 16 und ggf. der Temperatursensor 30 in eine gemeinsame Messkammer integriert sein.

Der WL-Detektor 16 umfasst typischerweise einen Metallblock mit mindestens zwei identisch aufgebauten Messzellen 32, 34. Eine dieser Messzellen 32 wird von dem zu analysierenden Gas, dem Probengas oder Messgas, durchströmt, die andere Messzelle 34 wird dauerhaft von dem umströmenden Messgas isoliert und stattdessen von einem reinen Gas mit bevorzugt bekannter und gleichbleibender Zusammensetzung durchströmt. Diese gegenüber dem Messgasvolumen abgeschlossene Messzelle 34 dient zur Vergleichsmessung.

In beiden Messzellen 32, 34 befindet sich jeweils ein Heizdraht (auch Filament genannt) aus z. B. Platin, Wolfram, Nickel oder deren Legierungen, der auf eine höhere Temperatur geheizt wird als der ihn umgebende Detektorblock. Es findet daher ein kontinuierlicher Wärmestrom von den Heizdrähten durch die umhüllenden Gasströme zum Detektorblock statt, der von der Wärmeleitfähigkeit (und damit von der Zusammensetzung) der Gase abhängig ist. Änderungen in der Zusammensetzung des Messgases verursachen daher Temperaturänderungen in der Messzelle 32 und damit eine Änderung des elektrischen Widerstands in den Heizdrähten. Das Messprinzip beruht somit auf der kontinuierlichen Messung der Wärmeleitfähigkeitsdifferenz des Probengasstroms gegenüber einem Referenzgasstrom.

Indem Mess- und Referenzzelle 32, 34 zu einer Wheatstone'schen Brückenschaltung zusammengeschlossen sind, lassen sich die Temperaturdifferenzen der Heizdrähte als Spannungsunterschied messen und aufzeichnen. Dabei bilden die Heizdrähte der beiden Messzellen 32, 34 zwei nach Art eines Spannungsteilers angeordnete elektrische Widerstände, die über einen dazwischen liegenden Brückenzweig mit einem parallel geschalteten Spannungsteiler abgeglichen werden. Das so gemessene Detektorsignal ist dabei in erster Näherung der Probenkonzentration, hier z. B. der Konzentration von $H_2$ im Messgas proportional.

Bei der in FIG. 1 dargestellten Variante sind insgesamt vier Messzellen 32, 32a, 34, 34a in der Messkammer 12 angeordnet, die paarweise als Mess- und Referenzzelle zusammenwirken. In jedem der beiden Spannungsteilerstränge, die über eine der Diagonalen mit Betriebsspannung versorgt und in der anderen Diagonalen (im Brückenzweig) über eine Spannungsmesseinheit gegeneinander abgeglichen werden, ist also je eine Mess- und eine Referenzzelle angeordnet. Die Spanungsmesseinheit ist dabei in Gestalt eines Wärmeleitfähigkeit-Auswertungsmoduls 36 in die außerhalb des Containments 4 angeordnete Auswerteinheit 26 integriert.

In einer nicht dargestellten alternativen Variante mit insgesamt zwei Messzellen 32, 34 kann der Spannungsteiler, der dem aus Mess- und Referenzzelle gebildeten Spannungsteiler gegenüber liegt, aus zwei Festwiderständen gebildet sein, die insbesondere in die Auswerteinheit 36 ausgelagert sein können.

Der WT-Detektor 14 arbeitet ebenfalls in an sich bekannter Weise:
Durch die Membran 28 der Messkammer 10, insbesondere in Gestalt einer porösen Sintermetallscheibe, gelangt Messgas in die Messkammer 10. Darin befinden sich zwei durch Widerstandsdrähte gebildete Heizelemente, die durch Anlegen einer Heizspannung auf eine Arbeitstemperatur von etwa 500 bis 600 °C erhitzt werden. Eines der Heizelemente, der Pellistor 40, ist an der Oberfläche mit einem Katalysator 42 beschichtet, so dass eventuell vorhandener Wasserstoff ($H_2$) bei gleichzeitiger Anwesenheit von Sauerstoff ($O_2$) katalytisch zu Wasserdampf ($H_2O$) verbrannt bzw. flammenlos rekombiniert wird. Die daraus resultierende Temperaturerhöhung führt zu einer Steigerung des elektrischen Widerstandes im Heizdraht des Pellistors 40. Das zweite Heizelement, der Kompensator 44, ist mit einer chemisch inerten Schicht überzogen und dient als Referenzwiderstand zum Pellistor 40. Umgebungseinflüsse wie Temperatur und Luftfeuchtigkeit, welche zu einer Veränderung der Temperatur der Heizelemente führen, werden auf diese Weise kompensiert.

Die bei der Rekombinationsreaktion auftretende Differenz der Widerstandswerte der von Pellistor 40 und Kompensator 44 gebildeten Messzellen 46, 48 wird ähnlich wie bei dem WL-Detektor 16 über eine Wheatstone'sche Messbrücke gemessen. Bei der in FIG. 1 dargestellten Variante sind dazu die beiden Festwiderstände 50, 52 des zweiten Spannungsteilerstrangs in die externe Auswerteeinheit 26 ausgelagert. Die Messung der Brückenspannung erfolgt in dem Wärmetönungs-Auswertungsmodul 54.

Eine autarke Strom- / Spannungsversorgungseinheit 56, die insbesondere nach dem Prinzip einer Unterbrechungsfreien Stromversorgung (USV) ausgestaltet ist, übernimmt die Strom- / Spannungsversorgung der Brückenschaltungen und der Auswertungsmodule.

Über eine Zuleitung 90 kann zu Test- und Kalibrierungszwecken ein Testgas mit wohldefinierter, bekannter Zusammensetzung in die Messkammern 10, 12 eingeleitet werden.

Die im Wärmeleitfähigkeit-Auswertungsmodul 36 und Wärmetönungs-Auswertungsmodul 56 erfassten Brückenspannungen werden beispielsweise direkt in diesen Modulen und/oder in einem nachgeschalteten Messdatenverarbeitungsmodul 58 in der weiter unten beschriebenen Weise verarbeitet.

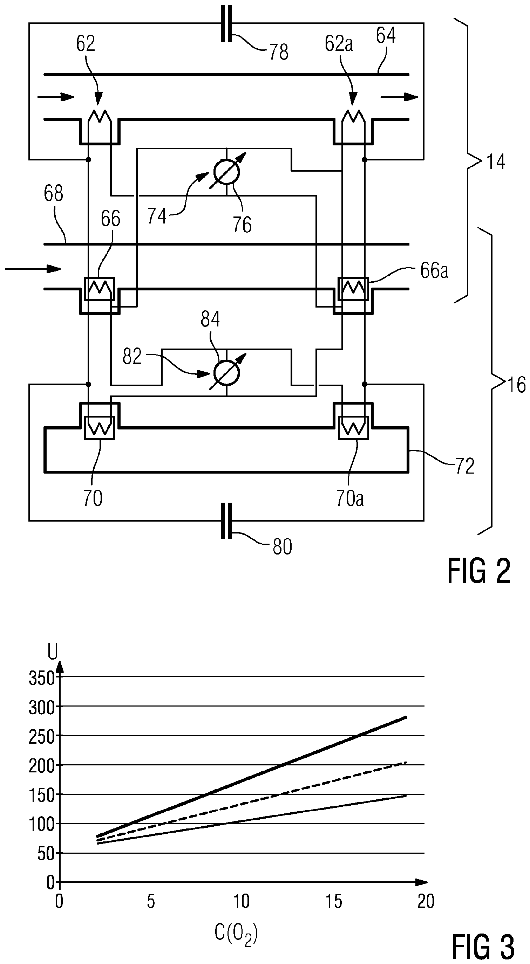

Eine besonders vorteilhafte Variante einer Widerstands-Brückenschaltung, bei der der WT-Detektor 14 und der WL-Detektor 16 in einen gemeinsamen Schaltkreis mit zwei Messbrücken integriert sind, ist in FIG. 2 schematisch dargestellt.

Die duale Brückenschaltung 60 umfasst zwei gleichartige katalytisch aktive Messzellen 62, 62a, die von dem Messgas um- bzw. durchströmt werden, was schematisch durch den Strömungskanal 64 angedeutet ist. Sie umfasst weiterhin zwei gleichartige katalytisch inaktive Messzellen 66, 66a, die ebenfalls von dem Messgas um- bzw. durchströmt werden, was schematisch durch den Strömungskanal 68 angedeutet ist. Alternativ können die Messzellen auch in einem gemeinsamen Strömungskanal für das Messgas angeordnet sein, sofern durch einen geeignet gewählten Mindestabstand ein thermisches Übersprechen der aktiven zu den inaktiven Messzellen vermieden ist. Schließlich gibt es noch zwei gleichartige, katalytisch inaktive Messzellen 70, 70a, die von dem Messgas isoliert sind, was schematisch durch die Anordnung in einem gegenüber der Umgebung abgeschlossenen und insbesondere mit einem Referenzgas gefüllten Vergleichsvolumen 72 angedeutet ist.

Die Verdrahtung ist so gewählt, dass die katalytisch inaktiven, vom Messgas umströmten Messzellen 66, 66a in der Mitte als Referenzzellen sowohl für die katalytisch aktiven, vom Messgas umströmten Messzellen 62, 62a oben als auch für die katalytisch inaktiven, gegenüber dem Messgas abgeschlossenen Messzellen 70, 70a unten zur Verfügung stehen. Dabei bilden die katalytisch inaktiven, vom Messgas umströmten Messzellen 66, 66a in der Mitte einerseits zusammen mit den katalytisch aktiven, vom Messgas umströmten Messzellen 62, 62a oben einen Detektor nach dem Wärmetönungsprinzip (WT-Detektor 14), und andererseits zusammen mit den katalytisch inaktiven, gegenüber dem Messgas abgeschlossenen Messzellen 70, 70a unten einen Detektor nach dem Wärmeleitungsprinzip (WL-Detektor 16). Die Bezeichnungen "oben, unten, in der Mitte" dienen selbstverständlich nur zur leichteren Erfassbarkeit der Zeichnung und beinhalten keine Einschränkung für die tatsächliche räumliche Lage der Messzellen.

Konkret bildet je eine katalytisch aktive, vom Messgas umströmte Messzelle (z. B. 62) mit der ihr kreuzweise gegenüber liegenden, katalytisch inaktiven und vom Messgas umströmten Messzelle (z. B. 66a) einen Spannungsteiler des WT-Detektors 14, der über den Brückenzweig 74 an den anderen der beiden Spannungsteiler angekoppelt ist. In den Brückenzweig 74 ist ein Spannungsmessgerät 76 zur Messung der WT-Brückenspannung geschaltet. Die Spannungsversorgung der Brückenschaltung wird durch die Spannungsquelle 78 bewerkstelligt.

Für den im spiegelbildlich verschalteten WL-Detektor 16 mit den Messzellen 66, 66a und 70, 70a gelten die obigen Ausführungen analog. Hier übernimmt die Spannungsquelle 80 die Spannungsversorgung der Messbrücke, und in den Brückenzweig 82 ist ein Spannungsmessgerät 84 zur Messung der WL-Brückenspannung geschaltet.

Ein wesentlicher Vorteil dieser Schaltung gegenüber anderen denkbaren Varianten besteht darin, dass weniger Kabel zu den Sensoren geführt werden müssen.

Aufgrund der vergleichsweise geringen Sensitivität des WL-Detektors 16 wird er im vorliegenden Fall bevorzugt zur Messung der Konzentration von $H_2$ im oberen Messbereich von > 10 Vol.-% bis 30 Vol.-% bis hin zu 100 Vol.-% eingesetzt (sogenannter Störfallweitbereich).

Darüber hinaus kann der WL-Detektor 16 auch für eine grobe Messung der $H_2$-Konzentration im unteren Messbereich < 10 Vol.-% einsetzt werden, insbesondere für eine vergleichende Messung und einen Konsistenzabgleich mit dem WT-Detektor 14.

Insbesondere kann eine Messung der $H_2$-Konzentration im Messgas mit Hilfe des WL-Detektors 16 erfolgen, wenn nur wenig oder gar kein Sauerstoff im Messgas vorhanden ist. Dies ist möglich, da die Funktion des WL-Detektors 16 wie oben beschrieben nicht von der Anwesenheit von $O_2$ im Messgas abhängt. Eine derartige Situation mit Sauerstoffmangel ist verlässlich mit Hilfe des WT-Detektors 14 erkennbar, da die dort normalerweise erfolgende katalytische Rekombination auf einen ausreichenden Sauerstoffgehalt im Messgas angewiesen ist.

Schließlich dient die vom WL-Detektor 16 gemessene $H_2$-Konzentration als Eingangsgröße für eine katalytische unterstützte Bestimmung der $O_2$-Konzentration im Zusammenspiel mit dem WT-Detektor 14, wie weiter unten beschrieben.

Der WT-Detektor 14 dient bevorzugt zur Messung der $H_2$-Konzentration im unteren Messbereich < 10 Vol.-% bei ausreichendem, vorzugsweise mindestens stöchiometrischem $O_2$-Gehalt zur Einleitung und Aufrechterhaltung der Rekombinationsreaktion. Damit sind gerade in der Anfangsphase eines schweren Störfalls eine sichere und schnelle Erkennung einer $H_2$-Freisetzung und eine Überwachung des entsprechenden Konzentrationsverlaufs ermöglicht.

Da die Effizienz der $H_2$/$O_2$-Rekombination und demzufolge auch die vom WT-Detektor 14 gemessene Brückenspannung vom Gehalt beider Gasanteile im Messgas abhängen, ist es prinzipiell möglich, mit Hilfe des WT-Detektors 14 auch die $O_2$-Konzentration zu bestimmen.

Bei hinreichend hohen $H_2$-Konzentrationen von z. B. > 10 Vol.-% ist dies auf direktem Wege möglich auf der Basis von zuvor empirisch durch Versuchsreihen ermittelten und ggf. theoretisch fundierten Kennlinien der Brückenspannung in Abhängigkeit von der $H_2$-Konzentration einerseits und von der $O_2$-Konzentration andererseits. Zusammengefasst erfolgt die $O_2$-Konzentrationsmessung in dem genannten $H_2$-Hochkonzentrationsregime durch Kalibrierung des Messsignals der katalytischen Reaktion am WT-Detektor 14.

Dies wird beispielhaft in FIG. 3 veranschaulicht: Dort ist schematisch die am WT-Detektor 14 gemessene Brückenspannung U in mV als Funktion der $O_2$-Konzentration $C(O_2)$ in Vol.-% aufgetragen, und zwar für drei verschiedene $H_2$-Konzentrationen (10, 20 und 30 Vol.-%) als Parameter der Kurvenschar. In dem dargestellten Intervall ist der Kurvenverlauf annähernd linear. Bei bekannter $H_2$-Konzentration, insbesondere durch den Messwert des WL-Detektors 16, lässt sich damit aus der gemessenen Brückenspannung die $O_2$-Konzentration eindeutig aus der Kurvenschar ablesen (ggf. durch Interpolieren).

Ähnliche Kennlinien lassen sich für die Brückenspannung als Funktion der $H_2$-Konzentration mit der $O_2$-Konzentration als Scharparameter ermitteln.

Bei geringen $H_2$-Konzentrationen von z. B. < 2,5 Vol.-% und dementsprechend verminderter katalytischer Reaktion erfolgt die $O_2$-Konzentrationsbestimmung indirekt und semianalytisch durch Messung der Gesamtwärmeleitfähigkeit des Messgases mit dem WL-Detektor 16 und durch Auflösung der Einzelleitfähigkeiten und Gaskonzentrationen unter ergänzenden Annahmen - insbesondere durch Bestimmung der Dampfkonzentration über Temperaturmessung unter der Annahme von Sattdampfbedingungen.

Die Auswertung beruht in diesem Fall beispielsweise auf folgenden Annahmen:
Wenn man davon ausgeht, dass im Containment zunächst Luft (air) bei Umgebungstemperatur von 293 K vorhanden ist, und dann zu Beginn eines Störfalles zunächst im Wesentlichen Wasserstoff ($H_2$) und Wasserdampf ($H_2O$) freigesetzt werden, dann gelten unter der Annahme von Massenerhaltung im Containment für die Konzentrationen C und die Drücke p die Beziehungen: (1)

$$C(total) = C(air) + C(H_2) + C(H_2O) = 1$$

(2)

$$p(total) = p(air) + p(H_2) + p(H_2O)$$

Die anfänglich vorhandene Luft setzt sich im Wesentlichen aus Sauerstoff ($O_2$) und Stickstoff ($N_2$) zusammen,

wobei sich die Sauerstoffkonzentration im Containment im Verlauf des Störfalls durch Oxidationsreaktionen ändert, während der Stickstoff inert ist und nicht am Reaktionsgeschehen teilnimmt. Sein Anteil an der anfänglich vorhandenen Luft beträgt bekanntlich rund 79 %: (3)

$$C(N_2) = 0,79\ C(air)$$

Die mit Hilfe des WL-Detektors 16 gemessene Gesamtwärmeleitfähigkeit des Messgases überlagert sich in guter Näherung additiv aus den Einzelleitfähigkeiten der Bestandteile $H_2$, $O_2$, $N_2$ und $H_2O$: (4)

$$\lambda(total) = \lambda(H_2)\ C(H_2) + \lambda(O_2)\ C(O_2) + \lambda(N_2)\ C(N_2) + \lambda(H_2O)\ C(H_2O)$$

Dabei bezeichnet $\lambda$ die aus der Literatur bekannten spezifischen Wärmeleitfähigkeiten.

Ferner lassen sich die Einzelkonzentrationen wie folgt durch die Partialdrücke der einzelnen Bestandteile ausdrücken: (5)

$$C(H_2O) = p(H_2O)\ /\ p(total)$$

(6)

$$C(H_2) = p(H_2)\ /\ p(total)$$

(7)

$$C(H_2O) = p(H_2O)\ /\ p(total)$$

Der Partialdruck der Luft lässt sich mit Hilfe des idealen Gasgesetzes $p = k\ T$ wie folgt approximieren: (8)

$$p(air) = 1\ bar * T\ /\ 293\ K$$

Dabei bezeichnet T die Temperatur des Messgases, die über den Temperatursensor 30 gemessen wird. Die Konstante k wurde auf den Umgebungsdruck von 1 bar bei Umgebungstemperatur 293 K (= 20 °C) normiert.

Wenn man ferner Sattdampfbedingungen (Gleichgewichtsdampfdruck bei Gleichgewicht von flüssiger und dampfförmiger Phase) unterstellt, dann lässt sich der Partialdruck des Wasserdampfes $H_2O$ aus Standard-Tabellenwerken als Funktion der gemessenen Temperatur nachschlagen: (9)

$$p(H_2O) = p_{steam}(T)$$

Setzt man nun die Gleichungen (1) bis (3) und (5) bis (9) in die Gleichung (4) ein und löst nach der Sauerstoffkonzentration auf, so erhält man einen Ausdruck, der nur von den direkt messbaren Größen $\lambda(total)$, $C(H_2)$ und T und von den aus der Literatur bekannten Parametern $\lambda(H_2)$, $\lambda(O_2)$, $\lambda(N_2)$, $\lambda(H_2O)$ sowie den ebenfalls bekannten Sättigungs-Dampfdruckkurven von Wasserdampf $H_2O$ abhängt: (10)

$$C(O_2) = F(\lambda(total),\ C(H_2),\ T\ |\ \lambda(H_2),\ \lambda(O_2),\ \lambda(N_2),\ \lambda(H_2O))$$

Auf diese Weise lässt sich unter den getroffenen Annahmen $C(O_2) > C(H_2)$ und $C(H_2) \approx\ < 2,5$ Vol.-% die Sauerstoffkonzentration $C(O_2)$ aus dem Messwert $\lambda(total)$ des WL-Detektors 16, dem mit einem ähnlichen Kalibrierverfahren wie oben beschrieben erhaltenen Messwert $C(H_2)$ des WT-Detektors 14 und dem mit Hilfe des Temperatursensors 30 erhaltenen Messwert für die Temperatur T des Messgases ermitteln.

Eine Standard-Fehlerrechnung in Gaußscher Approximation unter der zusätzlichen Annahme, dass der wesentliche Fehlerbeitrag von der gemessenen $H_2$-Konzentration herrührt, ergibt für den Messfehler (Standardabwei-

chung): (11)

$$\Delta C(O_2) \approx (\lambda(H_2) / \lambda(O_2)) \Delta C(H_2) \approx 7 \Delta C(H_2)$$

Damit ist der ermittelte Wert für die $O_2$-Konzentration zwar unvermeidbar mit einem Fehler behaftet, der rund 7-mal höher ist als der Messfehler für die $H_2$-Konzentration. Unter den getroffenen, durch die tatsächlichen Verhältnisse in der Anfangsphase eines schweren Störfalls gerechtfertigten Annahmen ist dies gleichwohl eine vernünftige Approximation.

Die oben beschriebenen Auswertungen und Berechnungen werden zweckmäßigerweise automatisiert in dem Messdatenauswertungsmodul 58 ausgeführt, welches durch entsprechende Hardware und/oder Software für diese Aufgabe ertüchtigt ist.

Bevorzugt erfolgt dabei ein Konsistenzabgleich der von beiden Detektoren 14 und 16 gelieferten Messwerte und eine automatische Auswahl der unter den gegeben Umständen geeignetsten Auswertungsmethode anhand der oben genannten Kriterien.

Wie sich der obigen Beschreibung entnehmen lässt, wird im Bereich kleiner $H_2$-Konzentrationen < 10 Vol.-% die Bestimmung der $O_2$-Konzentration nach der Methode der katalytischen Wärmetönung oder durch die Kombination von Wärmeleitfähigkeit und katalytischer Wärmetönung ungenauer, insbesondere im Messbereich hoher $O_2$-Konzentrationen > 15 Vol.-%. Dies kann insbesondere dann passieren, wenn der gesamte $H_2$-Gehalt in der Atmosphäre - jedenfalls in der Umgebung des Sensorkopfes - durch die katalytische Reaktion umgesetzt ist.

Um die $O_2$-Konzentration auch bei derartigen Verhältnissen zuverlässig und mit vergleichsweise hoher Genauigkeit bestimmen zu können, wenn sich nur wenig oder gar kein Wasserstoff in der Atmosphäre befindet, ist vorteilhafterweise zusätzlich zu den WL/WT-Messzellen eine Zirkoniumdioxid-Messzelle 92 in den Sensorkopf integriert. Das für die Messung benötigte Referenzgas (vorzugsweise Luft) befindet sich hierbei in einer zur Umgebung hermetisch dichten Sensorkopfkammer. Eine beispielsweise mittels eines elektrischen Heizelementes beheizte Zirkoniumdioxid-Membran 94 trennt das Referenzgas von der Containmentatmosphäre.

Die Funktionsweise ist die Folgende: Ab einer Temperatur von rund 650 °C treten in Zirkoniumdioxid ($ZrO_2$) folgende grundlegende physikalische Mechanismen auf:

- $ZrO_2$ sondert Sauerstoff-Ionen ab und wird zu einem Festkörperelektrolyt für Sauerstoff. Durch Anlegen einer Stromquelle kann umgebender Sauerstoff durch das $ZrO_2$ transportiert werden.
- Da das $ZrO_2$ bei den genannten Temperaturen sich wie ein Elektrolyt verhält, kann eine galvanische Spannung (sogenannte Nernst-Spannung) gemessen werden, wenn an den Grenzflächen des $ZrO_2$-Elements eine Sauerstoffdruckdifferenz vorliegt.

Damit ist es möglich, mit Hilfe der beheizten $ZrO_2$-Messzelle 92 den Sauerstoffgehalt in der umgebenden Atmosphäre relativ zum Referenzgas auf potentiometrische Weise zu ermitteln. Das entsprechende Messsignal wird über die zusätzliche Signalleitung 96 der Auswerteeinheit 26 zugeführt und dort ausgewertet, mithin der $O_2$-Gehalt in der Messatmosphäre bestimmt.

Damit ist in dem genannten Bereich kleiner $H_2$-Konzentrationen (< 10 Vol.-%) die $ZrO_2$-basierte Messung als redundante Messmethode wirksam, und es können hohe Genauigkeiten über den gesamten $O_2$-Messbereich (üblicherweise 0 bis 25 Vol.-%) erzielt werden.

Bei $H_2$-Konzentrationen > 10 Vol.-% kann es bei ungünstigen $O_2$-Konzentrationen durch die beheizte $ZrO_2$-Messung bei den genannten Temperaturen zu einer spontanen Umsetzung des $H_2$-Gehaltes kommen (Knallgasreaktion, Entzündung). Zur Vermeidung dieses Prozesses wird die $ZrO_2$-basierte Messung bei höheren $H_2$-Gehalten von > 10 Vol.-% von der gemeinsamen Auswerteelektronik ausgeschaltet, insbesondere das Heizelement für die Beheizung der $ZrO_2$-Membran 94 deaktiviert. Der hierfür benötigte Messwert der $H_2$-Konzentration wird bevorzugt von der Wärmeleitfähigkeitsmessung geliefert. Im Bereich von $H_2$-Konzentrationen > 10 Vol.-% wird dann die $O_2$-Konzentrationsmessung wie weiter oben beschrieben durch die kombinierte WL/WT-Messung vollzogen.

Auch wenn die Messvorrichtung 2 hier bislang ausschließlich im Zusammenhang mit einer Überwachung der Atmosphäre in einem Containment eines Kernkraftwerks beschrieben wurde, so ist doch klar, dass sie auch in anderen kerntechnischen Anlagen einsetzbar ist, etwa in einem Zwischenlager oder in einer Aufbereitungsanlage. Darüber hinaus ist eine Anwendung in jeglicher Industrieanlage denkbar, in der mit der Freisetzung von Wasserstoff und Sauerstoff zu rechnen ist und dementsprechend die Gefahr einer Knallgasexplosion besteht. Das Messprinzip lässt sich schließlich auch auf andere Gase übertragen, die sich katalytisch miteinander rekombinieren lassen.

Bezugszeichenliste

| | |
|---|---|
| 2 | Messvorrichtung |
| 4 | Containment |
| 6 | Kernkraftwerk |
| 8 | Sicherheitshülle |
| 10 | Messkammer |
| 12 | Messkammer |
| 14 | WT-Detektor |
| 16 | WL-Detektor |
| 18 | Anschlusskammer |
| 20 | Anschlussleitungen |
| 22 | Anschlussstecker |
| 24 | Kabeldurchführung |
| 26 | Auswerteeinheit |
| 28 | Membran |
| 30 | Temperatursensor |
| 32, 32a | Messzelle |
| 34, 34a | Messzelle |
| 36 | WL-Auswertungsmodul |
| 40 | Pellistor |
| 42 | Katalysator |
| 44 | Kompensator |
| 46 | Messzelle |
| 48 | Messzelle |
| 50 | Festwiderstand |
| 52 | Festwiderstand |
| 54 | WT-Auswertungsmodul |
| 56 | Strom- / Spannungsversorgungseinheit |
| 58 | Messdatenauswertungsmodul |
| 60 | Brückenschaltung |
| 62, 62a | Messzelle |
| 64 | Strömungskanal |
| 66, 66a | Messzelle |
| 68 | Strömungskanal |
| 70, 70a | Messzelle |
| 72 | Vergleichsvolumen |
| 74 | Brückenzweig |
| 76 | Spannungsmessgerät |
| 78 | Spannungsquelle |
| 80 | Spannungsquelle |
| 82 | Brückenzweig |
| 84 | Spannungsmessgerät |
| 90 | Zuleitung |
| 92 | Zirkoniumdioxid-Messzelle |
| 94 | Zirkoniumdioxid-Membran |
| 96 | Signalleitung |

**Patentansprüche**

1. . Verfahren zur quantitativen Analyse der Zusammensetzung der Atmosphäre eines Containments (4) einer kerntechnischen Anlage, mittels einer Messvorrichtung (2), die

> • einen Wärmeleitfähigkeitsdetektor (16) mit einer ersten Messbrücke,
> • einen Wärmetönungsdetektor (14) mit einer zweiten Messbrücke, und

• eine gemeinsamen Auswerteeinheit (26)

umfasst, wobei in der Auswerteeinheit (26) anhand der an beiden Messbrücken anliegenden Brückenspannungen der Gehalt an Wasserstoff ($H_2$) und zugleich der Gehalt an Sauerstoff ($O_2$) bestimmt wird.

2. . Verfahren nach Anspruch 1, wobei in der Auswerteeinheit (26) anhand der an beiden Messbrücken anliegenden Brückenspannungen auf diversitäre Weise zwei Messwerte für den Gehalt an Wasserstoff ($H_2$) ermittelt werden.

3. . Verfahren nach Anspruch 1 oder 2, wobei bei Wasserstoffkonzentrationen von vorzugsweise > 10 Vol.-% der Gehalt an Sauerstoff ($O_2$) direkt aus einer empirisch ermittelten Kalibrierkurve für die Brückenspannung an der Messbrücke des Wärmetönungsdetektors (14) ermittelt wird.

4. . Verfahren nach einem der Ansprüche 1 bis 3, wobei bei Wasserstoffkonzentrationen von vorzugsweise < 2,5 Vol.-% die Sauerstoffkonzentration bestimmt wird aus

• der am Wärmeleitfähigkeitsdetektor (16) gemessenen Wärmeleitfähigkeit des Gasgemischs,
• der mittels Kalibrierung am Wärmetönungsdetektor (14) ermittelten Wasserstoffkonzentration des Gasgemischs, und
• der mit Hilfe eines Temperatursensors (30) ermittelten Temperatur des Gasgemischs,

wobei für die Auswertung der Dampfdruck von im Gasgemisch vorhandenem Wasserdampf unter der Annahme von Sättigung aus der Temperatur ermittelt wird.

5. . Verfahren nach einem der Ansprüche 1 bis 4, wobei bei Wasserstoff Konzentrationen von < 10 Vol.-% der Gehalt an Sauerstoff ($O_2$) auf redundante Weise durch Messung der an einer beheizten Zirkoniumdioxid-Messzelle (92) abgegriffenen galvanischen Spannung bestimmt wird.

6. . Verfahren nach Anspruch 5, bei dem die Beheizung der Zirkoniumdioxid- Messzelle (92) abgeschaltet wird, wenn die mit Hilfe des Wärmeleitfähigkeitsdetektors (16) und/oder Wärmetönungsdetektors (14) ermittelte Wasserstoffkonzentration > 10 Vol.-% ist.

7. . Messvorrichtung (2) zur quantitativen Analyse der Zusammensetzung der Atmosphäre eines Containments (4) einer kerntechnischen Anlage, welche

• einen Wärmeleitfähigkeitsdetektor (16) mit einer ersten Messbrücke,
• einen Wärmetönungsdetektor (14) mit einer zweiten Messbrücke, und
• eine gemeinsamen Auswerteeinheit (26)

umfasst, wobei die Auswerteinheit (26) für die Durchführung des Verfahrens nach einem der Ansprüche 1 bis 4 eingerichtet ist.

8. . Messvorrichtung (2) nach Anspruch 7, mit

• zwei vom Messgas umströmten, katalytisch aktiven Messzellen (62, 62a), die zusammen mit zwei vom Messgas umströmten, katalytisch inaktiven Zellen (66, 66a) die Messbrücke des Wärmetönungsdetektors (14) bilden,
• zwei vom Messgas isolierten, katalytisch inaktiven Messzellen (70, 70a), die zusammen mit den genannten zwei vom Messgas umströmten, katalytisch inaktiven Zellen (66, 66a) die Messbrücke des Wärmeleitfähigkeitsdetektors (16) bilden.

9. . Messvorrichtung (2) nach Anspruch 7 oder 8 mit einer beheizbaren Zirkoniumdioxid-Messzelle (92) für eine diversitar-redundante Bestimmung des Gehalts an Sauerstoff ($O_2$) in der Auswerteeinheit (26) bei Wasserstoffkonzentrationen von < 10 Vol.-%.

10. . Überwachungssystem für die Atmosphäre in einem Containment (4) eines Kernkraftwerkes (6) bei schweren Störfällen mit einer Messvorrichtung (2) gemäß einem der Ansprüche 7 bis 9.

**Claims**

1. A method for quantitatively analysing the composition of the atmosphere of a containment (4) of a nuclear installation, by means of a measuring device (2) comprising

    • a thermal conductivity detector (16) with a first measuring bridge,
    • a heat tint detector (14) with a second measuring bridge, and
    • a common evaluation unit (26)

    whereby the hydrogen ($H_2$) content and simultaneously the oxygen ($O_2$) content are determined in the evaluation unit (26) on the basis of the bridge voltages applied to both measuring bridges.

2. A method according to claim 1, whereby two measured values for the content of hydrogen ($H_2$) are determined in the evaluation unit (26) in a diverse manner using the bridge voltages applied to both measuring bridges.

3. A method according to claim 1 or 2, whereby at hydrogen concentrations of preferably > 10% in volume, the oxygen ($O_2$) content is determined directly from an empirically determined calibration curve for the bridge voltage at the measuring bridge of the heat tint detector (14).

4. A method according to one of claims 1 to 3, whereby at hydrogen concentrations of preferably < 2.5% in volume the oxygen concentration is determined from

    - the thermal conductivity of the gas mixture measured at the thermal conductivity detector (16),
    - the hydrogen concentration of the gas mixture determined by calibration on the heat tint detector (14), and
    - the temperature of the gas mixture determined with the aid of a temperature sensor (30),

    whereby the vapour pressure of water vapour present in the gas mixture is determined from the temperature for the evaluation, assuming saturation.

5. A method according to any one of claims 1 to 4, whereby at hydrogen concentrations of < 10% in volume, the content of oxygen ($O_2$) is determined in a redundant manner by measuring the galvanic voltage tapped at a heated zirconia measuring cell (92).

6. A method according to claim 5, in which the heating of the zirconium dioxide measuring cell (92) is switched off when the hydrogen concentration determined with the aid of the thermal conductivity detector (16) and/or heat tint detector (14) is > 10% in volume.

7. A measuring device (2) for quantitatively analysing the composition of the atmosphere of a containment (4) of a nuclear installation, comprising

    • a thermal conductivity detector (16) with a first measuring bridge,
    • a heat tint detector (14) with a second measuring bridge, and
    - a common evaluation unit (26)

    wherein the evaluation unit (26) is set up for carrying out the method according to one of claims 1 to 4.

8. A measuring device (2) according to claim 7, with

    - two catalytically active measuring cells (62, 62a) around which the measuring gas flows and which, together with two catalytically inactive cells (66, 66a) around which the measuring gas flows, form the measuring bridge of the heat tint detector (14),
    - two catalytically inactive measuring cells (70, 70a) isolated from the measuring gas, which together with the two catalytically inactive cells (66, 66a) around which the sample gas flows form the measuring bridge of the thermal conductivity detector (16).

9. A measuring device (2) according to claim 7 or 8 with a heatable zirconium dioxide measuring cell (92) for a diverse-redundant determination of the oxygen ($O_2$) content in the evaluation unit (26) at hydrogen concentrations of < 10 % in volume.

10. A monitoring system for the atmosphere in a containment (4) of a nuclear power plant (6) in the event of severe accidents, comprising a measuring device (2) according to one of claims 7 to 9.

**Revendications**

1. Procédé d'analyse quantitative de la composition de l'atmosphère d'une enceinte de confinement (4) d'une installation nucléaire, au moyen d'un dispositif de mesure (2) qui comprend

   - un détecteur de conductivité thermique (16) avec un premier pont de mesure,
   - un détecteur de chaleur de réaction (14) avec un deuxième pont de mesure, et
   - une unité d'évaluation (26) commune

   dans lequel la teneur en hydrogène ($H_2$) et en même temps la teneur en oxygène ($O_2$) sont déterminées dans l'unité d'évaluation (26) à l'aide des tensions de pont appliquées aux deux ponts de mesure.

2. Procédé selon la revendication 1, dans lequel deux valeurs de mesure pour la teneur en hydrogène ($H_2$) sont établies de manière diversifiée dans l'unité d'évaluation (26) sur la base des tensions de pont appliquées aux deux ponts de mesure.

3. Procédé selon la revendication 1 ou 2, dans lequel, pour des concentrations d'hydrogène de préférence > 10 % en volume, la teneur en oxygène ($O_2$) est établie directement à partir d'une courbe d'étalonnage déterminée empiriquement pour la tension de pont sur le pont de mesure du détecteur de chaleur de réaction (14).

4. Procédé selon l'une quelconque des revendications 1 à 3, dans lequel, pour des concentrations d'hydrogène de préférence < 2,5 % en volume, la concentration d'oxygène est déterminée à partir de

   - la conductivité thermique du mélange gazeux mesurée au niveau du détecteur de conductivité thermique (16),
   - la concentration d'hydrogène du mélange gazeux, établie au moyen d'un étalonnage sur le détecteur de chaleur de réaction (14), et
   - la température du mélange gazeux établie à l'aide d'un capteur de température (30),

   dans lequel la pression de vapeur de la vapeur d'eau présente dans le mélange gazeux est établie pour l'évaluation à partir de la température, en supposant une saturation.

5. Procédé selon l'une quelconque des revendications 1 à 4, dans lequel, pour des concentrations d'hydrogène < 10 % en volume, la teneur en oxygène ($O_2$) est déterminée de manière redondante par mesure de la tension galvanique prélevée sur une cellule de mesure en dioxyde de zirconium (92) chauffée.

6. Procédé selon la revendication 5, selon lequel le chauffage de la cellule de mesure en dioxyde de zirconium (92) est arrêté lorsque la concentration d'hydrogène établie à l'aide du détecteur de conductivité thermique (16) et/ou du détecteur de chaleur de réaction (14) est > 10 % en volume.

7. Dispositif de mesure (2) pour l'analyse quantitative de la composition de l'atmosphère d'une enceinte de confinement (4) d'une installation nucléaire, lequel comprend

   - un détecteur de conductivité thermique (16) avec un premier pont de mesure,
   - un détecteur de chaleur de réaction (14) avec un deuxième pont de mesure, et
   - une unité d'évaluation (26) commune

   dans lequel l'unité d'évaluation (26) est conçue pour la mise en oeuvre du procédé selon l'une quelconque des revendications 1 à 4.

8. Dispositif de mesure (2) selon la revendication 7, avec

   - deux cellules de mesure (62, 62a) catalytiquement actives, entourées par le gaz de mesure, qui forment avec deux cellules (66, 66a) catalytiquement inactives, entourées par le gaz de mesure, le pont de mesure du détecteur de chaleur de réaction (14),

- deux cellules de mesure (70, 70a) catalytiquement inactives, isolées du gaz de mesure, qui forment avec lesdites deux cellules (66, 66a) catalytiquement inactives, entourées par le gaz de mesure, le pont de mesure du détecteur de conductivité thermique (16).

9. Dispositif de mesure (2) selon la revendication 7 ou 8 avec une cellule de mesure en dioxyde de zirconium (92) pouvant être chauffée pour une détermination redondante diversifiée de la teneur en oxygène ($O_2$) dans l'unité d'évaluation (26) pour des concentrations en hydrogène < 10 % en volume.

10. Système de surveillance de l'atmosphère dans une enceinte de confinement (4) d'une centrale nucléaire (6) en cas d'accident grave avec un dispositif de mesure (2) selon l'une quelconque des revendications 7 à 9.

FIG 1

FIG 2

FIG 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006033160 A1
- JP S5770435 A